(19) Europäisches Patentamt
European Patent Office
Office européen des brevets

(11) Publication number: **0 160 513 B1**

(12) **EUROPEAN PATENT SPECIFICATION**

(45) Date of publication of the patent specification: **16.09.87**

(51) Int. Cl.⁴: **C 07 D 223/16**, A 61 K 31/55

(21) Application number: **85302892.6**

(22) Date of filing: **25.04.85**

(54) **Fenoldopam 4', 8-bis-hydrogen sulfates.**

(30) Priority: **26.04.84 US 604101**

(43) Date of publication of application: **06.11.85 Bulletin 85/45**

(45) Publication of the grant of the patent: **16.09.87 Bulletin 87/38**

(84) Designated Contracting States: **AT BE CH DE FR GB IT LI LU NL SE**

(56) References cited:
**GB - A - 1 592 434**
**US - A - 4 197 297**
**US - A - 4 388 240**

(73) Proprietor: **SMITHKLINE BECKMAN CORPORATION, P.O. Box 7929 1 Franklin Plaza, Philadelphia Pennsylvania 19101 (US)**

(72) Inventor: **Gaitanopoulos, Dimitri Eustratios, 1134 Bayless Place, Eagleville Pennsylvania 19403 (US)**
Inventor: **Weinstock, Joseph, 1234 Pothouse Road, Phoenixville Pennsylvania 19406 (US)**

(74) Representative: **Denerley, Paul Millington, Dr. et al, Smith Kline & French Laboratories Ltd Patent Department Mundells, Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

**Description**

This invention concerns new bis-sulfate derivatives of the renal dopaminergic agent, fenoldopam, and its optical isomers. More specifically, representative of the the compounds of this invention are 6-chloro-7,8-dihydroxy-1-(4'-hydroxyphenyl)-2,3,-4,5-tetrahydro-1H-3-benzazepine, 4',8-bis-hydrogen sulfate, R-6-chloro-7,8-dihydroxy-1-(4'-hydroxyphenyl)-2,3,4,5-tetrahydro-1H-3-benzazepine, 4',8-bis-hydrogen sulfate or their pharmaceutically acceptable salts.

*Description of the Prior Art*

U.S. Patent No. 4 388 240 describes the preparation and isolation of three monosulfate esters of fenoldopam. No mention is made of any disulfate esters.

*Description of the Invention*

The compounds of this invention have the following structural formula:

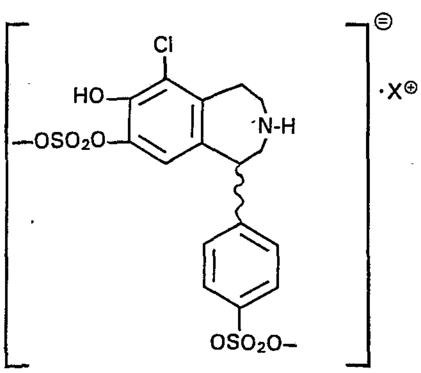

I

in which $X^{\oplus}$ is $H^{\oplus}$ or a pharmaceutically acceptable cation. The monoammonium, sodium or potassium salts are preferred.

The ammonium salts of the compounds of this invention (formula I in which $X^{\oplus}$ is $NH_4^{\oplus}$) is prepared by O-esterification of fenoldopam using a large excess of sulfur trioxide pyridine complex ($Py \cdot SO_3$) in a mixture of dry pyridine and dimethylformamide. The syrupy reaction mixture from the sulfation is purified using preparative high performance liquid chromatography over a $C_{18}$ reverse phase column using a mobile phase of 12% methanol/88% 0.05 N ammonium acetate buffer. The first fraction eluted from the column contains the 4',8-bis-hydrogen sulfate as the monoammonium salt. At least a stoichiometric quantity, but usually from 200-800% and preferably 500-600% excess, of sulfating agent is used.

The ammonium salt of fenoldopam is stable when purified but, upon neutralization, it slowly disproportionates into the 4' and 8-monosulfate esters of fenoldopam and fenoldopam itself. The free dibasic disulfate ester is prepared during the sulfation reaction and is converted to its relatively stable salt form during the work-up of the reaction mixture. The parent bis-hydrogen sulfate was not isolated.

The compounds of this invention are useful as a long-acting prodrug form of fenoldopam or its isomers, especially for parenteral use. In the anesthetized renal dog protocol, which is described in detail in U.S. Patent No. 4 197 297, the fenoldopam bis sulfate, ammonium salt, (formula 1 in which $X^{\oplus}$ is $NH_4^{\oplus}$) had a renal vascular resistance $Ed_{15}$ of 69 µg/kg during the test period of intravenous infusion but a potent 100% increase in renal blood flow was observed over a lengthy post test period. The renal dopaminergic activity lasted for two hours without a significant change in heart rate or blood pressure.

While the mono-8-sulfate ester of fenoldopam had an $ED_{15}$ of 19 µg/kg in this test, no drug effect was observed in the post-drug period of the protocol. Therefore, the bis-sulfate was a more effective pro-drug for fenoldopam than was the monosulfate form.

The 4',8-bis-hydrogen sulfate of the R-Isomer of fenoldopam also demonstrated a long-lasting biological effect. This compound at 100 µg/kg/min infusion for 24 hours in rodents did not cause arterial lesions as did fenoldopam itself at a lower dosage level.

The salts of formula I are particularly useful to prepare parenteral products which produce biological utilities similar to those of the parent compound but over an extended period. Exemplary of the uses of the compounds are for treatment of hypertension, congestive heart failure or severe kidney dysfunction. The compounds of this invention are administered intravenously by infusion using an effective, nontoxic dose selected from the range of 0.02-1.5 mcg/kg/min for a human patient of average weight. An effective dosage unit of from 5-35 mg is administered from 1-5 times daily for intramuscular use.

Pharmaceutical dosage unit preparatives are prepared using extenders such as mannitol, preservatives or inert buffers to maintain isotonic properties. Freeze dried or sterile dispersible powders in pouches, dosage unit ampoules or multidose vials are typical parenteral carrier forms. These are administered intravenously or intramuscularly as needed to induce an antihypertensive effect in a human patient in need thereof. Oral dosage units containing from 75-500 mg of the ester combined with a solid carrier in the form of a capsule, or tablet are prepared by methods known to the art. These are administered from 1-3 times daily to a patient in need of dopaminergic treatment but are usually less effective than are the parenteral product forms.

Other pharmaceutically acceptable salts are included in this invention. Certain alkali metal, alkaline earth metal or organic amine salts are known to the art to be so used. These are prepared as described herein, by forming the mono-salts prior to purification. Among the preferred cations of this group (that is, $X^{\oplus}$ of formula 1) are sodium or potassium. These are more stable than are the ammonium salt counterparts. It should be noted that one group is usually internally neutralized by the basic center at position 3.

The following example is intended to illustrate this invention. All temperatures are Centigrade.

*Example 1*

*Sulfur trioxide pyridine complex:*

Chlorosulfonic acid (3.0 ml, 0.0454 mol, 150 M%) was added slowly to a solution of dry pyridine (9.84 ml, 0.121 mol) in dry dimethylformamide (60 ml) which had been cooled to −10°. The resulting clear solution was stirred for 10 minutes at room temperature.

*O-Sulfation of fenoldopam:*

The freshly prepared pyridine complex solution was added all at once to a stirred solution of fenoldopam as the methane sulfonate salt (12.06 g, 0.030 mol, 100 M%) in dry pyridine (180 ml). The mixture was stirred for 0.5 hours at room temperature, then, heated on a steam bath for 0.5 hours. The resulting dark amber solution was chilled for 0.5 hours at −10°, filtered to remove pyridinium salts, transferred into a tared 1-necked, 2-L, round bottom flask and evaporated on a Buchi rotavap. At 70°, at reduced pressure, the excess pyridine complex began to sublime (0.5 hr) and a syrupy residue was formed. At this point, the cold-traps were cleaned and the syrupy residue was rotavaped at 70°, at reduced pressure, for 45 minutes to remove most of the volatiles, thereby leaving 23.83 g of syrup. The syrup should weigh no more than 26.91 g — the thereoretical weight of the total solid content of the reaction mixture.

The syrup was dissolved in 90 ml of mobile phase (MP) [12% methanol, 88% buffer (0.05 N ammonium acetate, pH 4)]. The flask was scratched to induce crystallization, then, left standing at room temperature for 3 hours. The resulting crystalline solid was filtered, washed with 50 ml of the mobile phase mixture and dried to give 6.41 g of solid which is mostly 4'-sulfate. The combined filtrate and washing (140 ml) contains 7.0 g of O-sulfates and traces of fenoldopam. The pH is adjusted to pH 3.4-4.0 with hydrochloric acid or aqueous ammonia as needed.

*Chromatography:*

The partially purified reaction mixture was further purified by preparative reverse phase high performance liquid chromatography (HPLC).

*Preparative HPLC*

| | |
|---|---|
| Apparatus: | JY-100 CHROMATOSPAC |
| Column: | 40 mm × 45 cm |
| Solid Phase (SP): | Whatman, Partisil 40 ODS-3 (192 g) length 37 cm (Lot # 100627) |
| Mobile Phase (MP): | 12% methanol<br>88% buffer (0.05N $NH_4OAc$, pH 4) |
| Flow Rate: | 50 ml/min |
| Pressure Rate: | 2.0 bar |
| Detector: | GOW-MAC 80-850 LC/LV preparative detector 280 nm |
| Attenuation: | AUFS 0.64 |

The sample (140 ml) was injected at 5-10 ml/min. Then, the flow rate was adjusted to 50 ml/min.

The first fraction, containing the 4',8-bis ester, eluted between 18 and 26 min (k' = 3.46) (500 ml); 4'-$SO_3$ eluted between 38 and 48 min (k' = 6.88) (700 ml); and the fraction containing 7-$SO_3$/8-$SO_3$ (23:75%) mixture eluted between 60-85 min (k' = 11.50) (1000-1400 ml).

*Isolation of fenoldopam 4',8-bis-hydrogen sulfate*

The 4',8-bis-hydropgen sulfate was purified by concentrating the designated fraction on a rotavap at 50°, aspirator pressure, then lyophilizing. The resulting solid residue crystallized as the ammonium salt upon trituration with methanol. The product was washed with methanol and air dried to give an analytically pure ammonium hydrate of fenoldopam 4',8-bis-hydrogen sulfate, mp, 160° softens, 180° melts, 185° resolidifies, 200-205° dec.

*Analytical*

The preparative reactions and chromatographic fractions were analyzed by analytical HPLC.

| | |
|---|---|
| Column: | WHATMAN, Partisil 5 ODS-3 RAC II-10 |
| Mobile Phase: | same as preparative HPLC |
| Flow Rate: | 2 mil/min |
| Pressure: | $13.8X10^{-6}$Pa |
| Detector: | 275 nm |

| *Compound* | *Retention (min)* | *k'* |
|---|---|---|
| 4',8-di$SO_3$ | 2.63 | 3.46 |
| 4'-$SO_3$ | 3.93 | 5.66 |
| 7-$SO_3$ | 6.16 | 9.44 |
| 8-$SO_3$ | 6.88 | 10.66 |
| fenoldopam | 7.83 | 12.27 |

Anal. Calcd. for $C_{16}H_{16}ClNO_9S_2 \cdot NH_3 \cdot 2.5\ H_2O$: C, 36.40; H, 4.58; N, 5.31. Found: C, 37.00; H, 4.40; N, 5.30.

*Example 2*

Pyridine sulfur trioxide (23.71 g, 0.149 mol, 600 M%) was added all at once to a stirred solution of (9.98 g, 0.024 mol, 100 M%) of R-6-chloro-7,8-dihydroxy-1-(4'-hydroxyphenyl)-2,3,4,5-tetrahydro--1H-3-benzazepine, methyl sulfonate salt (the R-isomer of fenoldopam) in a mixture of 50 ml of dried dimethylformamide and 100 ml of dried pyridine at room temperature under argon gas. The reaction mixture was heated on the steam bath for 0.5 hour, then chilled for 1 hour on an acetone-ice bath. The deliquescent solid which separated was removed by filtration. The filtrate was evaporated to leave 35 g of syrup (sample A).

This material was dissolved in 40 ml of water (pH 2-3) from which it began to crystallize. Dilution to 100 ml and standing in the cold overnight gave a solid which was separated, washed carefully with cold water and methanol to give 4.52 g (39%) of crude bis-sulfate.

*Analysis of the reaction fractions:*

High pressure liquid chromatography (HPLC) over a cellulose column («Partisil 5») using 12% methanol, 88% buffer (0.05 N ammonium acetate, pH 4).

I. Sample A (syrup)

| *Compound* | *Rf (min)* | *% [Conc.]* |
|---|---|---|
| Unknown 1 | 1.87 | 2.79 |
| Unknown 2 | 2.12 | 10.18 |
| 4',8-bis-$SO_3H$ | 2.67 | 69.06 |
| 4'-$SO_3H$ | 4.27; 4.89 | 3.07; 2.07 |
| 7-$SO_3H$ | 6.37 | 2.68 |
| 8-$SO_3H$ | 7.07 | 10.14 |
| Base | — | 0 |

II. Sample B (crude product)

| | | |
|---|---|---|
| Unknown 1 | — | 0 |
| Unknown 2 | — | 0 |
| 4',8-bis-$SO_3H$ | 2.62 | 86.91 |
| 4-$SO_3H$ | 4.80 | 2.0 |
| 8-$SO_3H$ | 6.95 | 11.8 |
| Base | — | 0 |

III. Sample 3 (crystallisation filtrate)

| | | |
|---|---|---|
| Unknown 1 | — | 0 |
| Unknown 2 | 2.08 | 16.73 |
| 4',8-bis-$SO_3H$ | 2.51 | 60.26 |
| 4-$SO_3H$ | 4.14; 4.73 | 5.05; 2.21 |
| 7-$SO_3H$ | 5.73; 6.18 | 1.01; 5.31 |
| 8-$SO_3H$ | 6.86 | 9.44 |

*Purification:*

The crude product (Sample B), 4.52 g, was suspended in 50 ml of 0.5 N ammonium acetate solution. Water was added to the suspension until all the solid had dissolved (~200 ml; pH 5). The solution was filtered and evaporated on a rotary evaporator. The resulting syrup was redissolved in water and lyophilized to give the product as the ammonium salt (4.92 g) which was 84.97% 4,8-bis-sulfate with 11.37% 8-sulfate.

This sample was purified over a preparative liquid chromatographic column using a cellulose («Partisil 40») column with a 12% methanol/88% 0.05 N ammonium acetate mobile phase and refractive index as an indicator. Fractions 11-21 were collected, evaporated to 20 ml, then lyophilized to give 3.54 g of product.

The product was purified using hot methanol (325 ml). The extract was filtered. The filtrate was concentrated to 40 ml on a rotary evaporate, then cooled to give 2.77 g of the desired 4',8-bis-hydrogen sulfate as the ammonium salt (100% pure by HPLC).

Anal. Calc'd for $C_{16}H_{16}ClNO_9S_2 \cdot NH_3 \cdot 0.5H_2O$: C, 39.06; H, 4.0; N, 5.69. Found: C, 39,06; H, 4,16; N, 5.56.

*Example 3*

R-6-Chloro-7,8-dihydroxy-1-(4'-hydroxyphenyl)-2,3,4,5-tetrahydro-1H-3-benzazepine-4',8-bis-hydrogen sulfate, ammonium salt (2.48 g, 0.005 mol, prepared as in Example 2) was dissolved in 20 ml of water. Sodium bicarbonate (0.423 g, 0.005 mole) was added to the mixture all at once. The mixture was stirred for 10 minutes, pH 4.5-5.0. The pale yellow solution was filtered and the filtrate lyophilized to give 2.65 g of the desired monosodium salt as a white solid. HPLC demonstrated 100% of the desired bis ester salt.

A 50 mg sample was recrystallized from 1 ml of methanol to give 40 mg of product; $[\alpha]_{1\%}$ $H_2O$ = +5.71°.

Anal. (Uncrystallized) Calcd. for $C_{16}H_{15}ClNNaO_9S_2 \cdot 2H_2O$: C, 36.68; H, 3.66; N 2.67; Na, 4.39. Found: C, 36.79; H, 3.61; N, 2.84; Na, 4.33.

Anal. (Crystallized) Calcd. for $C_{16}H_{15}ClNNaO_9S_2$ 1-1/3 $H_2O$: C, 37.54; H, 3.48; N, 2.74; Na, 4.49. Found: C, 37.51; H, 3.38; N, 2.69; Na, 4.76.

Samples of the ammonium salt of Example 2 and the sodium salt of the example were heated at 78° and 0.01 mm of mercury. The sodium salt was unchanged. The ammonium salt gave 64.3% unchanged with 23.2% as the 8-sulfate.

**Claims for the Contracting States:**
BE, CH, LI, UK, SE, IT, FR, DE, LU, NL

1. A compound of the formula:

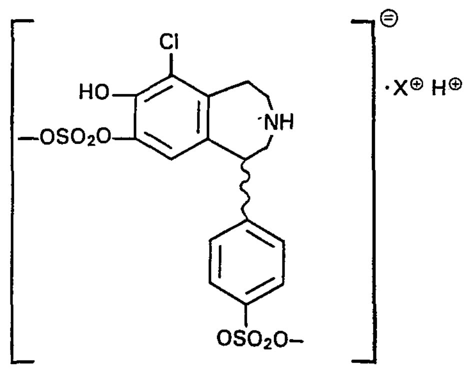

in which $X^{\oplus}$ is $H^{\oplus}$ or a pharmaceutically acceptable cation.

2. The compound according to claim 1 which is 6-chloro-7,8-dihydroxy-1-(4'-hydroxyphenyl)-2,3,4,5-tetrahydro-1H-3-benzazepine, 4',8-bis-hydrogen sulfate, ammonium salt.

3. The compound according to claim 1 which is 6-chloro-7,8-dihydroxy-1-(4'-hydroxyphenyl)-2,3,4,5-tetrahydro-1H-3-benzazepine, 4',8-bis-hydrogen sulfate, sodium salt.

4. The compound according to claim 1 which is R-6-chloro-7,8-dihydroxy-1-(4'-hydroxyphenyl)-2,3,4,5-tetrahydro-1H-3-benzazepine, 4',8-bis-hydrogen sulfate, sodium salt.

5. A pharmaceutical composition having renal dopaminergic activity comprising an effective therefor, nontoxic quantity of a compound according to one of claims 1-4 and a pharmaceutical carrier.

6. The composition of claim 5 in which the composition is adapted for parenteral use.

7. A compound according to one of claims 1-4 for pharmaceutical use.

8. The method of preparing a compound according to one of claims 1-4 comprising reacting a compound of the formula:

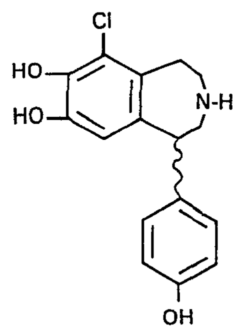

or a salt thereof, with two or more mole-equivalents of pyridine-sulfur trioxide, and, optionally, forming a salt of the product of said reaction.

**Claims for the Contracting State:** AT

1. A process for preparing a compound of the formula (I):

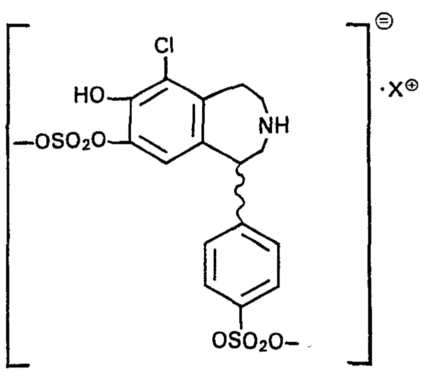

$\cdot X^{\oplus} H^{\oplus}$ (I)

wherein $X^{\oplus}$ is $H^{\oplus}$ or a pharmaceutically acceptable cation: which process comprises reacting a compound of the formula:

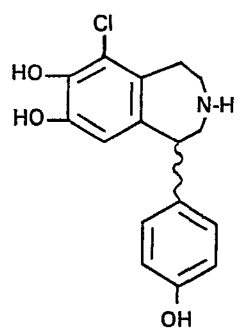

or a salt thereof, with two or more mole-equivalents of pyridine-sulfur trioxide, and optionally forming a salt of the product of said reaction.

2. A process according to claim 1 for preparing 6-chloro-7,8-dihydroxy-1-(4'-hydroxyphenyl)-2,3,4,5-tetrahydro-1H-3-benzazepine, 4',8-bis-hydrogen sulfate, ammonium salt.

3. A process according to claim 1 for preparing 6-chloro-7,8-dihydroxy-1-(4'-hydroxyphenyl)-2,3,4,5-tetrahydro-1H-3-benzazepine, 4',8-bis-hydrogen sulfate, sodium salt.

4. A process according to claim 1 for preparing R-6-chloro-7,8-dihydroxy-1-(4'-hydroxyphenyl)-2,3,4,5-tetrahydro-1H-3-benzazepine, 4',8-bis-hydrogen sulfate, sodium salt.

5. A process for preparing a pharmaceutical composition which comprises bringing into association a compound of the formula (I) or a pharmaceutically acceptable salt thereof as defined in any one of claims 1 to 4 and a pharmaceutically acceptable carrier.

**Patentansprüche für die Vertragsstaaten:** BE, CH, LI, GB, SE, IT, FR, DE, LU, NL

1. Eine Verbindung der Formel:

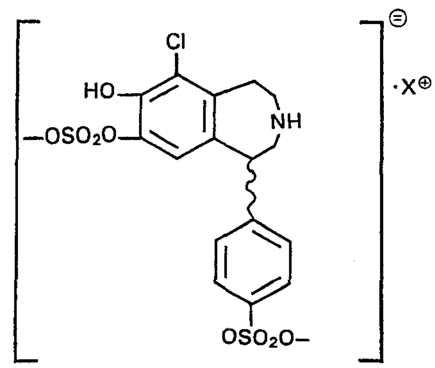

$\cdot X^{\oplus} H^{\oplus}$

in der $X^{\oplus}$ entweder $H^{\oplus}$ oder ein pharmazeutisch verträgliches Kation darstellt.

2. Verbindung nach Anspruch 1, nämlich 6-Chlor-7,8-dihydroxy-1-(4'-hydroxyphenyl)-2,3,4,5-tetrahydro-1H-3-benzazepin, 4',8-bis-hydrogensulfat-ammoniumsalz.

3. Verbindung nach Anspruch 1, nämlich 6-Chlor-7,8-dihydroxy-1-(4'-hydroxyphenyl)-2,3,4,5-tetrahydro-1H-3-benzazepin, 4',8-bis-hydrogensulfat-natriumsalz.

4. Verbindung nach Anspruch 1, nämlich R-6-Chlor-7,8-dihydroxy-1-(4'-hydroxyphenyl)-2,3,4,5-tetrahydro-1H-3-benzazepin, 4',8-bis-hydrogensulfat-natriumsalz.

5. Arzneimittel mit renal dopaminartiger Wirkung, umfassend eine wirksame, nicht toxische Menge einer Verbindung nach einem der Ansprüche 1 bis 4 und einen pharmazeutischen Träger.

6. Arzneimittel nach Anspruch 5, zubereitet für die parenterale Verwendung.

7. Eine Verbindung nach einem der Ansprüche 1 bis 4 zur pharmazeutischen Verwendung.

8. Verfahren zur Herstellung einer Verbindung nach einem der Ansprüche 1 bis 4 durch Umsetzung einer Verbindung der Formel:

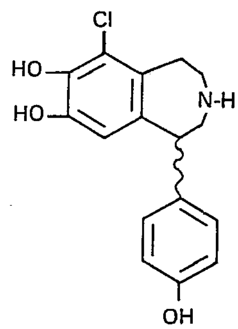

oder eines Salzes davon, mit einem oder zwei Moläquivalenten Pyridin-Schwefeltrioxid und gegebenenfalls Bildung eines Salzes dieses Reaktionsproduktes.

**Patentansprüche für den Vetragsstaat:** AT

1. Verfahren zur Herstellung einer Verbindung der Formel I:

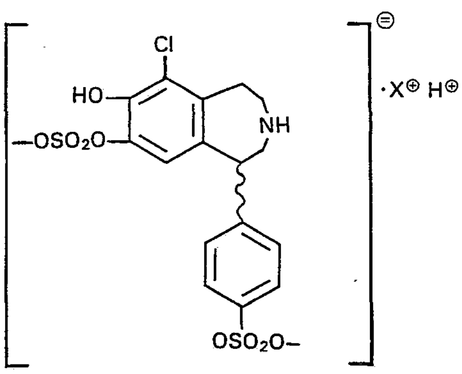

(I)

in der $X^{\oplus}$ entweder $H^{\oplus}$ oder ein pharmazeutisch verträgliches Kation darstellt, gekennzeichnet durch die Umsetzung einer Verbindung der Formel:

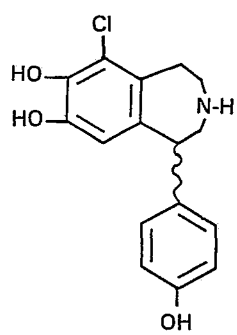

oder eines Salzes davon, mit einem oder zwei Moläquivalenten Pyridin-Schwefeltrioxid und gegebenenfalls die Bildung eines Salzes dieses Reaktionsproduktes.

2. Verfahren nach Anspruch 1 zur Herstellung des Ammoniumsalzes von 6-Chlor-7,8-dihydroxy-1-(4'-hydroxyphenyl)-2,3,4,5-tetrahydro-1H-3-benzazeptin, 4',8-bis-hydrogensulfat.

3. Verfahren nach Anspruch 1 zur Herstellung des Natriumsalzes von 6-Chlor-7,8-dihydroxy-1-(4'-hydroxyphenyl)-2,3,4,5-tetrahydro-1H-3-benzazepin, 4',8-bis-hydrogensulfat.

4. Verfahren nach Anspruch 1 zur Herstellung des Natriumsalzes von R-6-Chlor-7,8-dihydroxy-1-(4'-hydroxyphenyl)-2,3,4,5-tetrahydro-1H-3-benzazepin, 4',8-bis-hydrogensulfat.

5. Verfahren zur Herstellung eines Arzneimittels durch Zusammenbringen einer Verbindung der Formel I oder eines pharmazeutisch verträglichen Salzes davon nach einem der Ansprüche 1 bis 4 mit einem pharmazeutisch verträglichen Träger.

**Revendications pour les Etats contractants:**
BE, CH, DE, FR, GB, IT, LI, LU, NL, SE

1. Composé de la formule:

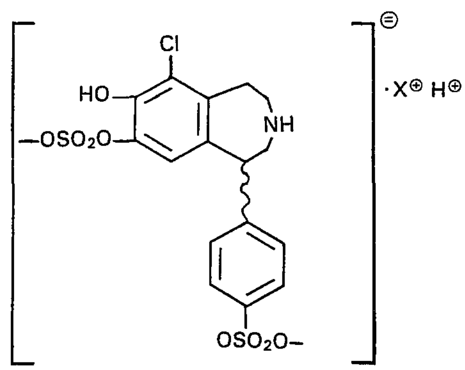

dans laquelle $X^{\oplus}$ est $H^{\oplus}$ ou un cation pharmaceutiquement acceptable.

2. Le composé selon la revendication 1 qui est 6-chloro-7,8-dihydroxy-1-(4'-hydroxyphényle)-2,3,4,5-tétrahydro-1H-3-benzazépine, 4',8-bis-hydrogène sulfate, le sel d'ammonium.

3. Le composé, selon la revendication 1, qui est 6-chloro-7,8-dihydroxy-1-(4'-hydroxyphényle)-2,3,4,5-tétrahydro-1H-3-benzazépine, 4',8-bis-hydrogène sulfate, le sel de sodium.

4. Le composé, selon la revendication 1, qui est R-6-chloro-7,8-dihydroxy-1-(4'-hydroxyphényle)-2,3,4,5-tétrahydro-1H-3-benzazépine, 4',8-bis-hydrogène sulfate, le sel de sodium.

5. Une composition pharmaceutique ayant une activité rénale dopaminergique, comprenant une quantité efficace du même, non toxique, d'un composé selon l'une des revendications de 1 à 4 et un support pharmaceutique.

6. La composition de la revendication 5 dans laquelle la composition est adaptée pour l'usage parentéral.

7. Un composé, selon l'une des revendications de 1 à 4 à l'usage pharmaceutique.

8. La méthode de préparation d'un composé selon l'une des revendications de 1 à 4 comprenant la mise en réaction d'un composé de la formule:

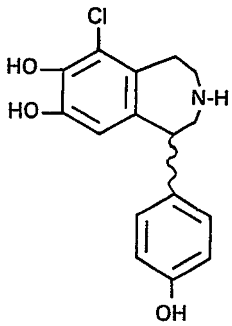

ou d'un sel du même, avec deux ou plusieurs équivalents-moles de trioxyde de pyridine-soufre, et, facultativement, la formation d'un sel du produit de ladite réaction.

**Revendications pour l'Etat contractant:** AT

1. Un procédé pour la préparation d'un composé de la formule (I):

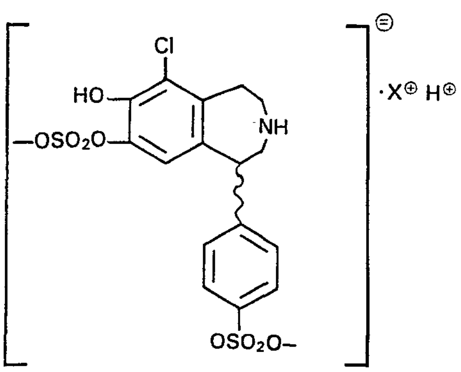

(I)

dans laquelle $X^{\oplus}$ est $H^{\oplus}$ ou un cation pharmaceutiquement acceptable: procédé qui comprend la mise en réaction d'un composé de la formule:

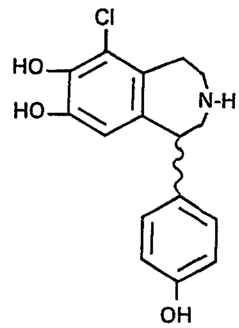

ou d'un sel du même, avec deux ou plusieurs équivalents-moles du trioxyde de pyridine-soufre, et facultativement, la formation d'un sel du produit de ladite réaction.

2. Un procédé selon la revendication 1 pour la préparation de 6-chloro-7,8-dihydroxy-1-(4'-hydroxyphényle)-2,3,4,5-tétrahydro-1H-3-benzazépine, 4',8-bis-hydrogène sulfate, le sel d'ammonium.

3. Un composé selon la revendication 1 pour la préparation de 6-chloro-7,8-dihydroxy-1-(4'-hydroxyphényle)-2,3,4,5-tétrahydro-1H-3-benzazépine, 4',8-bis-hydrogène sulfate, le sel de sodium.

4. Un procédé selon la revendication 1 pour la préparation de R-6-chloro-7,8-dihydroxy-1-(4'-hydroxyphényle)-2,3,4,5-tétrahydro-1H-3-benzazepine, 4',8-bis-hydrogène sulfate, le sel de sodium.

5. Un procédé pour la préparation d'une composition pharmaceutique qui comprend la mise en association d'un composé de la formule (I) ou d'un sel du même, pharmaceutiquement acceptable, comme défini dans n'importe laquelle des revendications de 1 à 4 et d'un support pharmaceutiquement acceptable.